# EUROPEAN PATENT SPECIFICATION

(11) **EP 3 951 658 B1**
(45) Date of publication and mention of the grant of the patent: **20.12.2023**
(21) Application number: 20305900.1
(22) Date of filing: 05.08.2020
(51) Int. Cl.: G06K 19/077

(54) **SENSOR ASSEMBLY AND SYSTEM**
SENSORANORDNUNG UND SYSTEM
ENSEMBLE CAPTEUR ET SYSTÈME

(43) Date of publication of application: 09.02.2022
(60) Divisional application: 23208227.1
(73) Proprietor: Becton Dickinson France, 38800 Le Pont-de-Claix (FR)
(72) Inventor: MARÉCHAL, Damien, 38640 Claix (FR); LE GAL-REDON, Patrick, 38170 Seyssinet Pariset (FR)
(74) Representative: Germain Maureau

(56) References cited:
- WO-A1-2006/002667
- DE-A1-102011 050 196
- US-A1- 2001 004 237

## Description

### BACKGROUND

### 1. Field

The present disclosure relates generally to a sensor assembly for an injection device and, in some non-limiting embodiments or aspects, to a sensory assembly for automatically capturing and communicating information related to use of the injection device.

### 2. Technical Considerations

A wide variety of injection devices for fluid injections are currently commercially available. However, many of these devices do not provide any mechanism to capture and/or transfer adherence monitoring data and/or other information related to use of the injection devices. WO 2006/002667 A1 is reflected in the preamble of claim 1.

### SUMMARY

The invention is set out in claim 1.

According to some non-limiting embodiments or aspects, provided is a sensor assembly for an injection device including: a disposable label configured to be attached to the injection device, the disposable label including: an electrically conductive trace, a first portion of the electrically conductive trace being configured to be altered in response to a first operation of the injection device; a reusable housing removably connected to the disposable label, the reusable housing including: a processor removably electrically coupled to the electrically conductive trace, the processor being configured to be activated in response to an alteration of the first portion of the electrically conductive trace; and a wireless communication device configured to wirelessly communicate, to a computing device, information associated with the alteration of the first portion of the electrically conductive trace.

According to some non-limiting embodiments or aspects, provided is a system including an injection device; and a sensor assembly including: a disposable label attached to the injection device, the disposable label including: an electrically conductive trace, a first portion of the electrically conductive trace being configured to be altered in response to a first operation of the injection device; a reusable housing removably connected to the disposable label, the reusable housing including: a processor removably electrically coupled to the electrically conductive trace, the processor being configured to be activated in response to an alteration of the electrically conductive trace; and a wireless communication device configured to wirelessly communicate, to a computing device, information associated with the alteration in the electrically conductive trace.

These and other features and characteristics of the present disclosure, as well as the methods of operation and functions of the related elements of structures and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of limits. As used in the specification and the claims, the singular form of "a," "an," and "the" include plural referents unless the context clearly dictates otherwise.

### BRIEF DESCRIPTION OF THE DRAWINGS

Additional advantages and details of embodiments or aspects of the present disclosure are explained in greater detail below with reference to the exemplary embodiments that are illustrated in the accompanying schematic figures, in which:
FIG. 1 is a diagram of non-limiting embodiments or aspects of an environment in which systems, devices, products, apparatus, and/or methods, described herein, may be implemented;
FIG. 2 is a perspective view of non-limiting embodiments or aspects of components of a sensor assembly of FIG. 1;
FIGS. 3A and 3B are perspective views of non-limiting embodiments or aspects of components of one or more devices and/or one or more systems of FIG. 1;
FIGS. 4A-4C are perspective views of non-limiting embodiments or aspects of components of one or more devices and/or one or more systems of FIG. 1;
FIG. 5 is a diagram of non-limiting embodiments or aspects of components of a sensor assembly of FIG. 1;
FIG. 6 is a diagram of an implementation of non-limiting embodiments or aspects of a sensor assembly;
FIG. 7 is a perspective view of an implementation of non-limiting embodiments or aspects of a sensor assembly;
FIG. 8 is a perspective view of an implementation of non-limiting embodiments or aspects of a sensor assembly; and
FIG. 9 is a perspective view of an implementation of non-limiting embodiments or aspects of a system including a sensor assembly.

### DETAILED DESCRIPTION

It is to be understood that the present disclosure may assume various alternative variations and step sequences, except where expressly specified to the contrary. It is also to be understood that the specific devices and processes illustrated in the attached drawings, and described in the following specification, are simply exemplary and non-limiting embodiments or aspects. Hence, specific dimensions and other physical characteristics related to the embodiments or aspects disclosed herein are not to be considered as limiting.

For purposes of the description hereinafter, the terms "end," "upper," "lower," "right," "left," "vertical," "horizontal," "top," "bottom," "lateral," "longitudinal," and derivatives thereof shall relate to the present disclosure as it is oriented in the drawing figures. However, it is to be understood that the present disclosure may assume various alternative variations and step sequences, except where expressly specified to the contrary. It is also to be understood that the specific devices and processes illustrated in the attached drawings, and described in the following specification, are simply exemplary embodiments or aspects of the present disclosure. Hence, specific dimensions and other physical characteristics related to the embodiments or aspects of the embodiments disclosed herein are not to be considered as limiting unless otherwise indicated.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." Furthermore, as used herein, the term "set" is intended to include one or more items (*e.g.,* related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least in partially on" unless explicitly stated otherwise.

As used herein, the terms "communication" and "communicate" refer to the receipt or transfer of one or more signals, messages, commands, or other type of data. For one unit (e.g., any device, system, or component thereof) to be in communication with another unit means that the one unit is able to directly or indirectly receive data from and/or transmit data to the other unit. This may refer to a direct or indirect connection that is wired and/or wireless in nature. Additionally, two units may be in communication with each other even though the data transmitted may be modified, processed, relayed, and/or routed between the first and second unit. For example, a first unit may be in communication with a second unit even though the first unit passively receives data and does not actively transmit data to the second unit. As another example, a first unit may be in communication with a second unit if an intermediary unit processes data from one unit and transmits processed data to the second unit. It will be appreciated that numerous other arrangements are possible.

It will be apparent that systems and/or methods, described herein, can be implemented in different forms of hardware, software, or a combination of hardware and software. The actual specialized control hardware or software code used to implement these systems and/or methods is not limiting of the implementations. Thus, the operation and behavior of the systems and/or methods are described herein without reference to specific software code, it being understood that software and hardware can be designed to implement the systems and/or methods based on the description herein.

Some non-limiting embodiments or aspects are described herein in connection with thresholds. As used herein, satisfying a threshold may refer to a value being greater than the threshold, more than the threshold, higher than the threshold, greater than or equal to the threshold, less than the threshold, fewer than the threshold, lower than the threshold, less than or equal to the threshold, equal to the threshold, etc.

As used herein, the term "computing device" or "computer device" may refer to one or more electronic devices that are configured to directly or indirectly communicate with or over one or more networks. The computing device may be a mobile device, a desktop computer, or the like. Furthermore, the term "computer" may refer to any computing device that includes the necessary components to receive, process, and output data, and normally includes a display, a processor, a memory, an input device, and a network interface. An "application" or "application program interface" (API) refers to computer code or other data sorted on a computer-readable medium that may be executed by a processor to facilitate the interaction between software components, such as a client-side front-end and/or server-side back-end for receiving data from the client. An "interface" refers to a generated display, such as one or more graphical user interfaces (GUIs) with which a user may interact, either directly or indirectly (e.g., through a keyboard, mouse, touchscreen, etc.).

As used herein, the term "server" may refer to or include one or more processors or computers, storage devices, or similar computer arrangements that are operated by or facilitate communication and processing for multiple parties in a network environment, such as the Internet, although it will be appreciated that communication may be facilitated over one or more public or private network environments and that various other arrangements are possible. Further, multiple computers, e.g., servers, or other computerized devices, such as POS devices, directly or indirectly communicating in the network environment may constitute a "system," such as a merchant's POS system. As used herein, the term "data center" may include one or more servers, or other computing devices, and/or databases.

As used herein, the term "mobile device" may refer to one or more portable electronic devices configured to communicate with one or more networks. As an example, a mobile device may include a cellular phone (e.g., a smartphone or standard cellular phone), a portable computer (e.g., a tablet computer, a laptop computer, etc.), a wearable device (e.g., a watch, pair of glasses, lens, clothing, and/or the like), a personal digital assistant (PDA), and/or other like devices. The terms "client device" and "user device," as used herein, refer to any electronic device that is configured to communicate with one or more servers or remote devices and/or systems. A client device or user device may include a mobile device, a network-enabled appliance (e.g., a network-enabled television, refrigerator, thermostat, and/or the like), a computer, and/or any other device or system capable of communicating with a network.

As used herein, the term "application" or "application program interface" (API) refers to computer code, a set of rules, or other data sorted on a computer-readable medium that may be executed by a processor to facilitate interaction between software components, such as a client-side front-end and/or server-side back-end for receiving data from the client. An "interface" refers to a generated display, such as one or more graphical user interfaces (GUIs) with which a user may interact, either directly or indirectly (*e.g.*, through a keyboard, mouse, etc.).

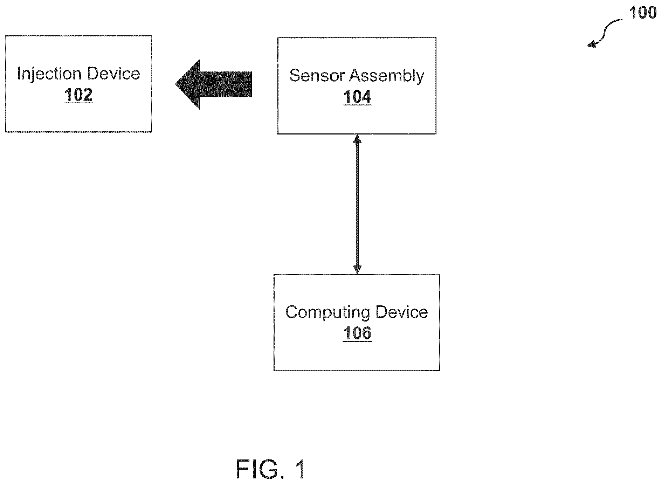

Referring to FIG. 1, non-limiting embodiments or aspects of an environment 100 in which systems, devices, products, apparatus, and/or methods, as described herein, may be implemented is shown. As shown in FIG. 1, environment 100 may include injection device 102, sensor assembly 104, and/or computing device 106. The number and arrangement of devices and systems shown in FIG. 1 is provided as an example. There may be additional devices and/or systems, fewer devices and/or systems, different devices and/or systems, or differently arranged devices and/or systems than those shown in FIG. 1. Furthermore, two or more devices and/or systems shown in FIG. 1 may be implemented within a single device and/or system, or a single device and/or system shown in FIG. 1 may be implemented as multiple, distributed devices and/or systems. Additionally, or alternatively, a set of devices and/or systems (e.g., one or more devices or systems) of environment 100 may perform one or more functions described as being performed by another set of devices and/or systems of environment 100.

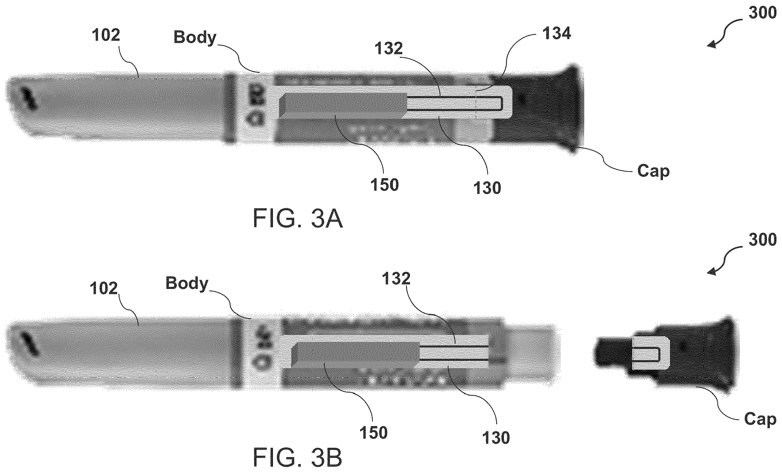

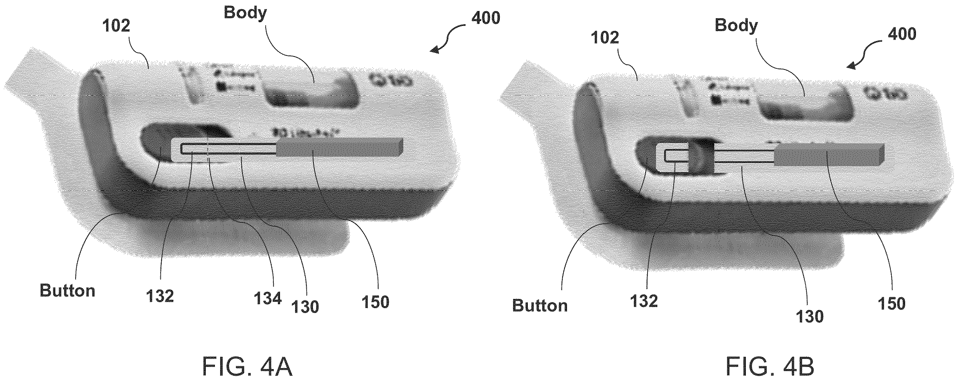

Injection device 102 may be configured to perform an injection operation to deliver a dose of medicament to a user. Injection device 102 may include injection devices with or without injection needles. Injection device 102 may be a disposable type injection device (e.g., a single use injection device, etc.) or a re-usable type injection device. Injection device 102 may include an auto injector, a pen injector, a wearable injector, and/or a passive needle guard. For example, injection device 102 may include the BD Intevia^{™} autoinjector, the BD Libertas^{™} wearable autoinjector, the BD Sonic^{™} autoinjector, and/or the BD UltraSafe Plus^{™} passive needle guard for a pre-filled ISO standard glass syringe. Further details regarding non-limiting embodiments or aspects injection device 102 are provided below with regard to FIGS. 3A, 3B, and 4A-4C.

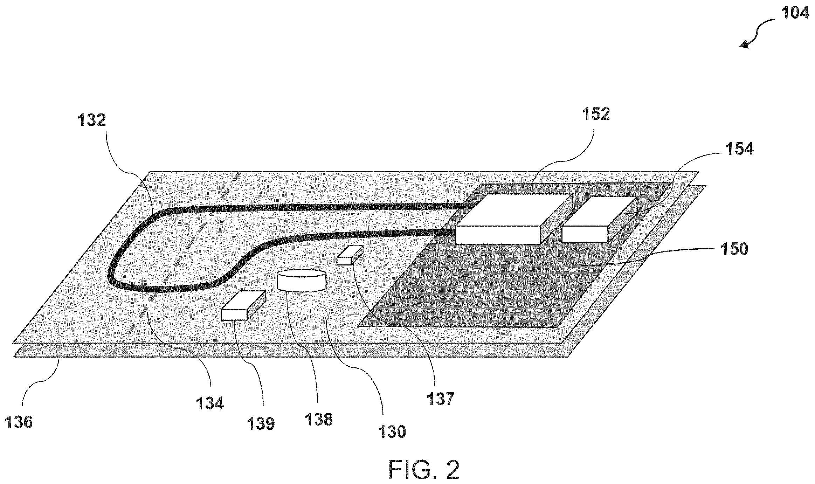

Referring also to FIG. 2, sensor assembly 104 may include disposable label 130 configured to be attached to injection device 102 and/or a reusable housing 150 removably connected to disposable label 130. Disposable label 130 may include a flexible and/or tear-able (or breakable) substrate. For example, disposable label 130 may be formed of paper, tamper evident tape, one or more polymers (e.g., PET, etc.), a flexible printed circuit board (PCB), or any combination thereof. Disposable label 130 may include an electrically conductive trace 132 (e.g., a circuit, etc.). For example, electrically conductive trace 132 may be on a surface of and/or located within (e.g., integrated with, housed within, not exposed by, etc.) disposable label 130. As an example, electrically conductive trace 132 may include a conductive material applied to disposable label 130 by at least one of the following processes: printing (e.g., an inkjet printing, screen printing, etc.), metal deposition and/or etching, flexography, or any combination thereof.

Portion 134 of disposable label 130 may be configured to be altered (e.g., bent, torn, broken, etc.). Alteration of portion 134 of disposable label 130 may cause a corresponding portion of electrically conductive trace 132 to be altered. For example, a first portion of electrically conductive trace 132 may be configured to be altered in response to an operation of injection device 102. As an example, portion 134 of disposable label 130 may be pre-cut, perforated, and/or the like, and tearing and/or breaking portion 134 of disposable label 130 may cause the corresponding portion of electrically conductive trace 132 to be torn and/or broken, thereby altering a state of a circuit formed by electrically conductive trace 132 (e.g., opening the circuit, closing the circuit, changing a resistance of the circuit, changing a voltage or a current flowing through the circuit, etc.). In such an example, a first portion of disposable label 130 including at least a portion of electrically conductive trace 132 may be attached to a removable and/or actuatable element of injection device 102 (e.g., a cap, a button, etc.) and a remainder of disposable label 130, which is attached to the first portion via portion 134, may be attached elsewhere to injection device 102, and removal and/or actuation of the removable and/or actuatable element of injection device 102 (e.g., a cap, a button, etc.) may cause the tearing and/or breaking of portion 134 of disposable label 130, thereby causing the corresponding portion of electrically conductive trace 132 to be torn and/or broken to alter the state of the circuit formed by electrically conductive trace 132.

FIGS. 3A and 3B are perspective views of an implementation 300 of non-limiting embodiments or aspects of sensor assembly 104 for injection device 102. As shown in FIG. 3A, a first portion of disposable label 130 including at least a portion of electrically conductive trace 132 may be attached to a cap of injection device 102 and a remainder of disposable label 130 may be attached to a body of injection device 102. As shown in FIG. 3B, removal of the cap of injection device 102 may cause the tearing and/or breaking of portion 134 of disposable label 130, thereby causing the corresponding portion of electrically conductive trace 132 to be torn and/or broken to alter the state of the circuit formed by electrically conductive trace 132.

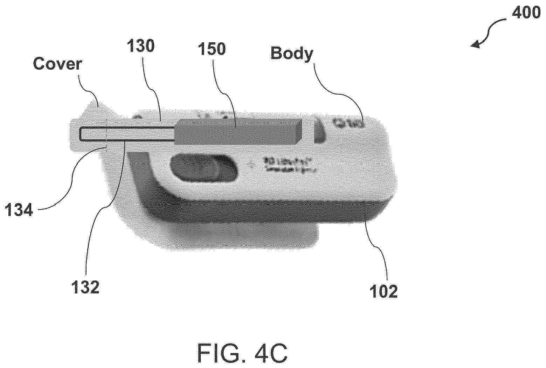

FIGS. 4A-4C are perspective views of an implementation 400 of non-limiting embodiments or aspects of sensor assembly 104 for injection device 102. As shown in FIG. 4A, a first portion of disposable label 130 including at least a portion of electrically conductive trace 132 may be attached to a button of injection device 102 and a remainder of disposable label 130 may be attached to a body of injection device 102. As shown in FIG. 4B, actuation of the button of injection device 102 may cause the tearing and/or breaking of portion 134 of disposable label 130, thereby causing the corresponding portion of electrically conductive trace 132 to be torn and/or broken to alter the state of the circuit formed by electrically conductive trace 132. FIG. 4C shows an alternative location of sensor assembly 104 where a first portion of disposable label 130 including at least a portion of electrically conductive trace 132 may be attached to a cover of injection device 102 and a remainder of disposable label 130 may be attached to a body of injection device 102. Removal of the cover of injection device 102 may cause the tearing and/or breaking of portion 134 of disposable label 130, thereby causing the corresponding portion of electrically conductive trace 132 to be torn and/or broken to alter the state of the circuit formed by electrically conductive trace 132.

In some non-limiting embodiments or aspects, disposable label 130 includes an adhesive layer 136. For example, adhesive layer 136 may adhere disposable label 130 to injection device 102 and/or a packing and/or container for sensor assembly 104. As an example, as shown in FIG. 2, electrically conductive trace 132 may be on a first face of disposable label 130 (e.g., a same face of disposable label 130 to which reusable housing 150 is attached, etc.), and adhesive layer 136 may be on a second face of disposable label 130 opposite the first face of disposable label 130.

In some non-limiting embodiments or aspects, disposable layer 130 includes sensor(s) 137. For example, sensor(s) 137 may include at least one of the following: an optical sensor (e.g. a printed photodetector, etc.), a temperature sensor, a force sensor, (e.g., a force resistive sensor, a membrane switch, etc.) or any combination thereof. As an example, sensor(s) 137 may be electrically coupled to the electrically conductive trace 132. In some non-limiting embodiments or aspects, sensor(s) 137 may be used instead of, or in addition to, portion 134 for detecting a removal and/or actuation of a removable and/or actuatable element of injection device 102 (e.g., a cap, a button, etc.). For example, a photodetector may be used to sense ambient light when a cap of injection device 102 is removed, a temperature sensor or force sensor may be used to sense a temperature change or a force applied to a surface (e.g., using force resistive sensors printed on label 130, a membrane switch on label 130, etc.) when a user actuates a button of injection device 102, and/or the like.

In some non-limiting embodiments or aspects, disposable label 130 includes battery 138 and/or memory 139. For example, battery 138 and/or memory 130 may be connected to electrically conductive trace 132. Battery 138 may provide power to components of reusable housing 150 (e.g., in response to the alteration of electrically conductive trace 132, etc.) when reusable housing 150 is attached to disposable label 130. Battery 138 may include a flexible battery, a thin film battery, a printed battery, or any combination thereof. Memory 139 may store a unique identifier associated with disposable label 130 and/or injection device 102. For example, the unique identifier may be accessed by and/or provided to components of reusable housing 150 (e.g., a processor, etc.) when reusable housing 150 is attached to disposable label 130 (e.g., in response to the alteration of electrically conductive trace 132, etc.).

Reusable housing 150 may include (e.g., carry, house, encompass, etc.) processor 152 and/or wireless communication device 154. Processor 152 may be removably electrically coupled to electrically conductive trace 132. In some non-limiting embodiments or aspects, processor 152 is configured to be activated in response to an alteration of a portion of electrically conductive trace 132 (e.g., in response to opening the circuit, closing the circuit, changing a resistance of the circuit, changing a voltage or a current flowing through the circuit, etc.). For example, the alteration of electrically conductive trace 132 may cause processor 152 to wake up, from a deep-sleep mode. As an example, a circuit formed by electrically conductive trace 132 may be intact in an initial state and operate as a closed switch, and when the user activates or operates injection device 102 and tears apart disposable label 130, electrically conductive trace 132 may be broken and operate as an open switch. However, non-limiting embodiments or aspects are not limited thereto, and a circuit formed by electrically conductive trace 132 may have as an initial state a closed circuit, an initial resistance, an initial voltage, an initial current, or any combination thereof and, after alteration, the circuit formed by electrically conductive trace 132 may have as a different state an open circuit, a second resistance, a second voltage, a second current, or any combination thereof. In some non-limiting embodiments or aspects, processor 152 and wireless communication device 154 are implemented in a single system-on-chip component.

Wireless communication device 154 may be configured to wirelessly communicate with computing device 106. For example, wireless communication device 154 may be configured to communicate information associated a use and/or operation of injection device 102 (e.g., information associated with an alteration of electrically conductive trace 132, sensor data, a unique identifier, etc.) to computing device 106. In some non-limiting embodiments or aspects, wireless communication device 154 includes one or more computing devices, chips, contactless transmitters, contactless transceivers, NFC transmitters, RFID transmitters, contact based transmitters, Bluetooth transceivers^{®} and/or the like that enables wireless communication device 154 to receive information directly from and/or communicate information directly to computing device 106 via a short range wireless communication connection (*e.g*., a communication connection that uses NFC protocol, a communication connection that uses Radio-frequency identification (RFID), a communication connection that uses a Bluetooth^{®} wireless technology standard, a communication protocol that uses a Zigbee wireless technology standard, a communication protocol that uses an Ant wireless technology standard, and/or the like).

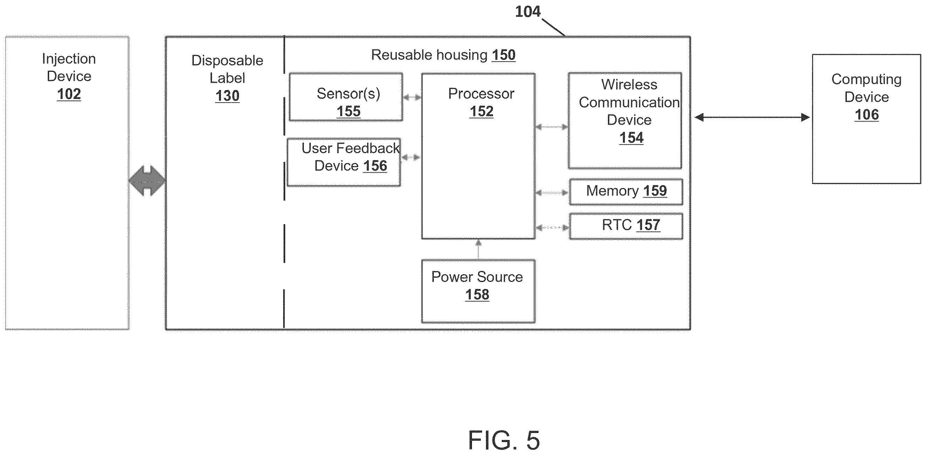

Referring now to FIG. 5, in some non-limiting embodiments or aspects, reusable housing 150 includes (e.g., carries, houses, encompasses, etc.) sensor(s) 155, user feed back device 156, RTC 157, power source 158, and/or member 159.

Sensor(s) 155 may include at least one of the following: a vibration sensor, an accelerometer, an optical sensor, a magnetic sensor, or any combination thereof. In some non-limiting embodiments or aspects, sensor(s) 155 is configured to detect a movement of a component of injection device 102 associated with an injection operation of injection device 102. For example, sensor 155 may be configured to detect a movement (e.g., a movement, a displacement, etc.) of at least one of a piston, a stopper of a plunger, a rod of the plunger, and a mobile trigger that initiates or triggers the movement of the plunger.

Processor 152 may be programmed and/or configured to determine, based on a state of electrically conductive trace 132, a signal or sensor data received from sensor(s) 137, and/or a signal or sensor data received from sensor(s) 155, information associated with a use and/or operation of injection device 102. In some non-limiting embodiments or aspects, information associated with a use and/or operation of injection device 102 includes at least one of the following: a time of removal of a cap of injection device 102, a time of removal of a cover of injection device 102, a time of actuation of a button of injection device 102, a start time of an injection operation, a duration of the injection operation, a speed of the injection operation, a back pressure profile of the injection operation, or any combination thereof. Processor 152 may store information associated with a use and/or operation of injection device 102 in memory 154.

User feedback device 156 may be configured to provide an indication associated with an injection operation and/or information associated a use and/or operation of injection device 102 to a user. For example, user feedback device 150 may include at least one of the following: a display, a light-emitting diode (LED), an audio output device (e.g., a buzzer, a speaker, etc.), or any combination thereof.

RTC 157 may include a computer clock (e.g., in the form of an integrated circuit, etc.) that keeps track of the current time. For example, processor 152 may time stamp information associated with a use and/or operation of injection device 102 based on the current time of RTC 157 (e.g., a cap removal time, a button actuation time, etc.).

Power source 158 may be configured to power processor 152, wireless communication device 154, sensor(s) 155, user feedback device 156, RTC 157, and/or memory 159. For example, power source 152 may include a battery (e.g., a rechargeable battery, a disposable battery, etc.).

Memory 159 may be configured to store information associated a use and/or operation of injection device 102. In some non-limiting embodiments or aspects, memory 159 stores a unique identifier associated with reusable housing 150 and/or injection device 102. For example, the unique identifier may be accessed by and/or provided to processor 152 in response to the alteration of electrically conductive trace 132.

Still referring to FIG. 5, and referring again to FIG. 1, computing device 106 may include one or more devices capable of receiving information and/or data from sensor assembly 104 and/or communicating information and/or data to sensor assembly 104. For example, computing device 106 may include a computing device, a server, a group of servers, a mobile device, a group of mobile devices, and/or the like. In some non-limiting embodiments or aspects, computing device 106 includes one or more computing devices, chips, contactless transmitters, contactless transceivers, NFC transmitters, RFID transmitters, contact based transmitters, and/or the like that enables computing device 106 to receive information directly from and/or communicate information directly to wireless communication device 154 via a short range wireless communication connection (*e.g*., a communication connection that uses NFC protocol, a communication connection that uses Radio-frequency identification (RFID), a communication connection that uses a Bluetooth^{®} wireless technology standard, a communication protocol that uses a Zigbee wireless technology standard, a communication protocol that uses an Ant wireless technology standard, and/or the like). In some non-limiting embodiments or aspects, computing device 106 may include and/or upload information and/or data to an electronic data management system, such as a hospital record system, a system used during a clinical trial to collect the trial related information, and/or the like.

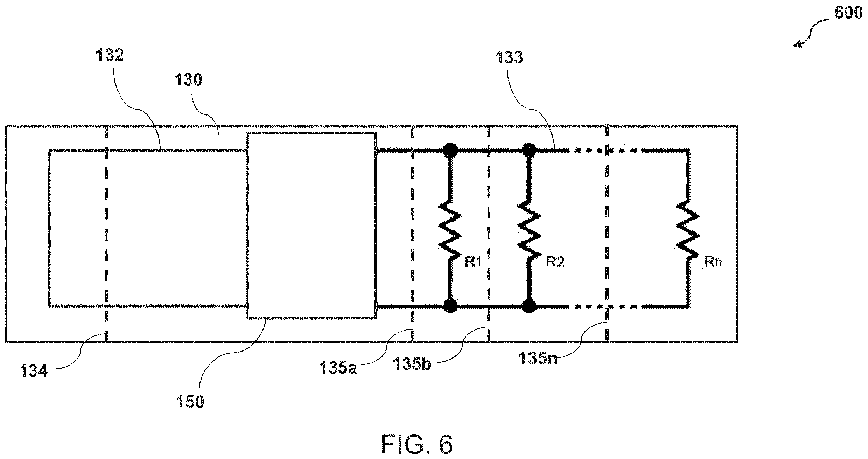

Referring now to FIG. 6, FIG. 6 is a diagram of an implementation 600 of non-limiting embodiments or aspects of sensor assembly 104. As shown in FIG. 6, disposable label 130 may include further electrically conductive trace 133, and processor 152 may be removably electrically coupled to the further electrically conductive trace. In some non-limiting embodiments or aspects, further electrically conductive trace may be the same as or similar to electrically conductive trace 132, except that a second portion of disposable label 130 including at least a portion of further electrically conductive trace 133 may be attached to a different removable and/or actuatable element of injection device 102 (e.g., a cap, a button, etc.) than the first portion of disposable label 130 including at least a portion of electrically conductive trace 132. As an example, a portion 135 of further electrically conductive 133 trace may be configured to be altered in response to a second operation of injection device 102 different than the first operation of injection device 102 in response to which the portion 134 of electrically conductive trace 132 is altered. As an example, the first operation of injection device 132 may include removal of a cap or a cover of injection device 102, and the second operation of injection device 102 may include actuation of a button of injection device 102 or a deployment of a needle shield of injection device 102.

Still referring to FIG. 6, in some non-limiting embodiments or aspects, a second portion of an electrically conductive trace (e.g., electrically conductive trace 132, further electrically conductive trace 133, etc.) is configured to be altered in response to a second operation of injection device 102 different than the first operation of the injection device in response to which a first portion of the electrically conductive trace is altered. For example, disposable label 130 may include portions 135a, 135b, ... 135n that are configured to be altered (e.g., bent, torn, broken, etc.) to cause a corresponding portion of further electrically conductive trace 133 to be altered, thereby altering a state of a circuit formed by further electrically conductive trace 133 (e.g., opening the circuit, closing the circuit, changing a resistance of the circuit, changing a voltage or a current flowing through the circuit, etc.). As an example, disposable label 130 may include a plurality of portions respectively including first resistor R1, second resistor R2, and third resistor R3 respectively connected to reusable housing 150 and each other via alterable portions 135a, 135b, and 135n. In such an example, a first portion of disposable label 130 including at least a portion of further electrically conductive trace 133 (e.g., including resistor Rn, etc.) may be attached to a removable and/or actuatable element of injection device 102 (e.g., a cap, a button, etc.) and another portion of disposable label 130, which is attached to the first portion via portion 135n (e.g., including resistor R2, etc.), may be attached elsewhere to injection device 102 (e.g., another removable and/or actuatable element of injection device 102, etc.), and removal and/or actuation of the removable and/or actuatable element of injection device 102 (e.g., the cap, the button, etc.) may cause the tearing and/or breaking of portion 135n of disposable label 130, thereby causing the corresponding portion of further electrically conductive trace 133 to be torn and/or broken to alter the state of the circuit formed by further electrically conductive trace 133, and removal and/or actuation of the another removable and/or actuatable element of injection device 102 may cause the tearing and/or breaking of portion 135b of disposable label 130, thereby causing the corresponding portion of further electrically conductive trace 133 to be torn and/or broken to alter the state of the circuit formed by further electrically conductive trace 133. For example, the first operation of injection device 102 may include removal of a cap or a cover of injection device 102, and the second operation of injection device 102 may include actuation of a button of injection device 102 or a deployment of a needle shield of injection device 102.

In some non-limiting embodiments or aspects, a further portion of disposable label 130, which is attached to the another portion via portion 135b (e.g., including resistor R1, etc.), may be attached elsewhere to injection device 102 (e.g., a further removable and/or actuatable element of injection device 102, etc.), and removal and/or actuation of the further removable and/or actuatable element of injection device 102 may cause the tearing and/or breaking of the portion 135a of disposable label 130, thereby causing the corresponding portion of further electrically conductive trace 133 to be torn and/or broken to alter the state of the circuit formed by further electrically conductive trace 133. As an example, an alteration caused to the state of the circuit may be different in response to tearing and/or breaking each of portions 135a, 135b, ... 135n (e.g., an alteration may open the circuit, close the circuit, change a resistance of the circuit, change a voltage or a current flowing through the circuit, etc.).

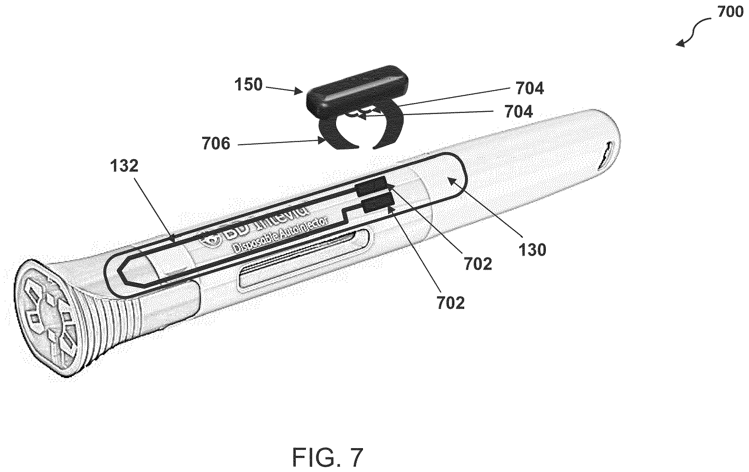

Referring now to FIG. 7, FIG. 7 is a diagram of an implementation 700 of non-limiting embodiments or aspects of sensor assembly 104. As shown in FIG. 7, disposable label 130 may include a plurality of first conductive pads 702 electrically coupled to electrically conductive trace 132 (and/or further electrically conductive trace 133), and reusable housing 150 may include a plurality of spring contacts 704 electrically coupled to processor 152 and removably electrically coupled to the plurality of first conductive pads 702. Reusable housing 150 may include a rigid deformable clip 706 configured to clip onto an exterior surface of injection device 102. For example, rigid deformable clip 706 may include a half-pipe shape, with a material forming the half-pipe shape being configured to deform around and grasp an exterior surface of injection device 102 when clip 706 is pressed onto injection device 102 to contact the plurality of first conductive pads 702 with the plurality of spring contacts 704. Reusable housing 150 may be attached to and/or integrated with rigid deformable clip 706. For example, reusable housing 150 may be attached to rigid deformable clip 706 via a clip, a snap fit, an adhesive, and/or other mechanical interface. Rigid deformable clip 706 may be configured and/or dimensioned according to a type (e.g., size, model, etc.) of injection device 102.

In some non-limiting embodiments or aspects, disposable label 130 includes a plurality of first conductive pads electrically coupled to electrically conductive trace 132 (and/or further electrically conductive trace 133), reusable housing 150 includes a plurality of second conductive pads coupled to processor 152 and removably electrically coupled to the plurality of first conductive pads via a conductive adhesive applied to at least one of the plurality of first conductive pads and the plurality of second conductive pads. For example, alternatively to, or in addition to, rigid deformable clip 706 and spring contacts 704 shown in FIG. 7, disposable label 130, reusable housing 150, the plurality of first conductive pads, and/or the plurality of second conductive pads may include an adhesive layer configured to hold the plurality of first conductive pads in contact with the plurality of second conductive pads (and/or disposable label 130 in contact with reusable housing 150).

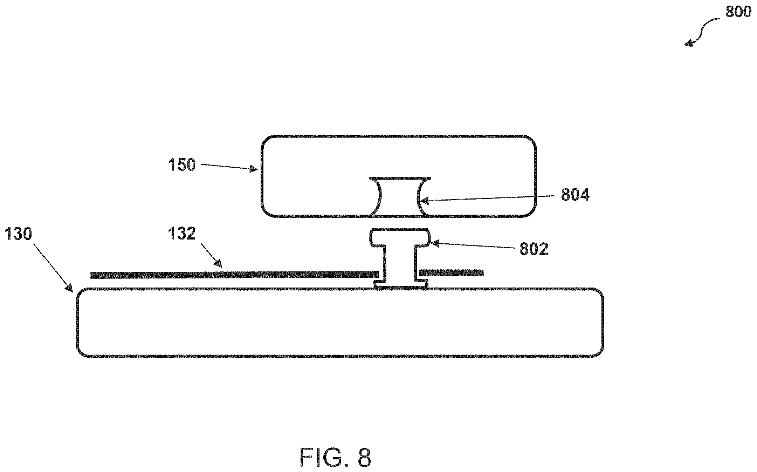

Referring now to FIG. 8, FIG. 8 is a diagram of an implementation 800 of non-limiting embodiments or aspects of sensor assembly 104. As shown in FIG. 8, disposable label 130 may include at least one connector 802 extending from disposable label 130, reusable housing 150 may include at least one port 804 in which the at least one connector 802 is received, and the at least one connector 802 may include a plurality of first electrical connectors (e.g., wires, pins, etc.) electrically coupled to electrically conductive trace 132 (and/or further electrically conductive trace 133) and removably electrically coupled to a plurality of second electrical connectors (e.g., wires, pins, etc.) of the at least one port 804, with the plurality of second electrical connectors 804 electrically coupled to processor 152. In some non-limiting embodiments or aspects, the at least one connector 802 is press fit in the at least one port 804.

In some non-limiting embodiments or aspects, the at least one connector 802 includes a plurality of connectors 802, and the at least one port 804 includes a plurality of ports 804 corresponding to the plurality of connectors 802. In some non-limiting embodiments or aspects, each connector of the plurality of connectors includes a single first electrical connector (e.g., a single wire, a single pin, etc.), and each port of the plurality of ports includes a single second electrical connector (e.g., a single wire, a single pin, etc.).

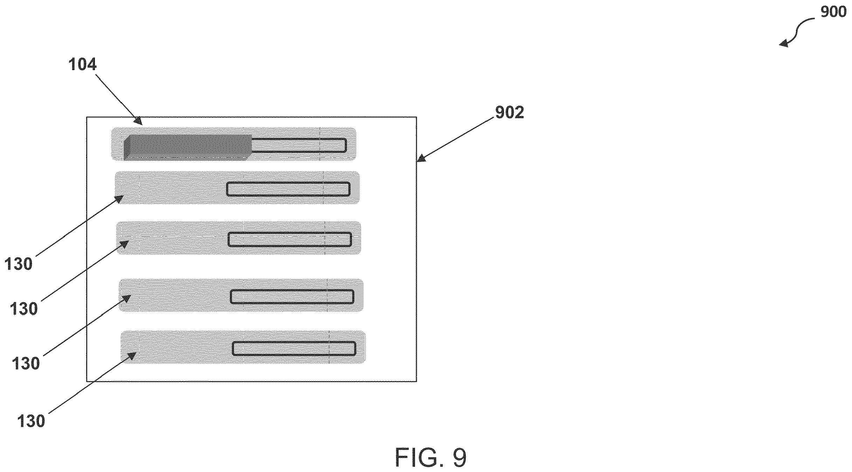

Referring now to FIG. 9, FIG. 9 is a diagram of an implementation 900 of non-limiting embodiments or aspects of a system including sensor assembly 104. As shown in FIG. 9, a system according to non-limiting embodiments or aspects may include injection device 102, sensor assembly(s) 104, and/or container 902. Disposable label 130 of sensor assembly 104 may include an electrically conductive trace (e.g., electrically conductive trace 132, further electrically conductive trace 133, etc.) with a portion of disposable label 130 including the electrically conductive trace attached to container 902. For example, a portion (e.g., portion 134, portion(s) 135a, 135b, ... 135n, etc.) of the electrically conductive trace may be configured to be altered in response to removal of sensor assembly 104 from container 902. As an example, reusable housing 150 may be reused with multiple disposable labels 130 and/or injection devices 102. For example, container 902 may include multiple disposable labels 130 and/or injection devices 102 and a reusable housing 150, and reusable housing 150 may be attached a different disposable label 130/injection device 102 each time a new disposable label 130/injection device 102 is removed from container 902. Accordingly, a cost may be lower than disposing of an entire module with each injection device, a pairing process between module and computing device 106, if any, may only be performed once, a user may be asked to bring back to a doctor (or a clinical research associate in the case of a clinical trial) only the reusable housing (avoiding manipulation of sharps) for reconciliation or adherence purposes, and any applicable recycling process requiring taking apart electronic components may be eased.

## Claims

1. A sensor assembly (104) for an injection device (102) comprising:
a disposable label (130) configured to be attached to the injection device (102), the disposable label (130) including:
an electrically conductive trace (132), wherein a first portion of the electrically conductive trace (132) is configured to be altered in response to a first operation of the injection device (102);
a reusable housing (150) removably connected to the disposable label (130), the reusable housing (150) including:
a processor (152) electrically coupled to the electrically conductive trace (132), wherein the processor (152) is configured to be activated in response to an alteration of the first portion of the electrically conductive trace (132); and
a wireless communication device (154) configured to wirelessly communicate, to a computing device, information associated with the alteration of the first portion of the electrically conductive trace (132),
**characterized in that**
the processor (152) is removably electrically coupled to the electrically conductive trace (132) and wherein the disposable label (130) includes a plurality of first conductive pads (702) electrically coupled to the electrically conductive trace (132), wherein the reusable housing (150) includes a plurality of spring contacts (704) electrically coupled to the processor (152) and removably electrically coupled to the plurality of first conductive pads (702), and wherein the reusable housing (150) includes a rigid deformable clip (706) configured to clip onto an exterior surface of the injection device (102).

2. The sensor assembly (104) of claim 1, wherein the disposable label (130) includes an adhesive layer (136).

3. The sensor assembly (104) of claim 1, wherein the disposable label (130) includes a battery (138).

4. The sensor assembly (104) of claim 1, wherein the reusable housing (150) includes a battery (138).

5. The sensor assembly (104) of claim 1, wherein the disposable label (130) includes a further electrically conductive trace (133), wherein a first portion of the further electrically conductive trace (133) is configured to be altered in response to a second operation of the injection device (102) different than the first operation of the injection device (102), and wherein the processor (152) is removably electrically coupled to the further electrically conductive trace (133).

6. The sensor assembly (104) of claim 5, wherein the first operation of the injection device (102) includes removal of a cap or a cover of the injection device (102), and wherein the second operation of the injection device (102) includes actuation of a button of the injection device (102) or a deployment of a needle shield of the injection device (102).

7. The sensor assembly (104) of claim 1, wherein a second portion of the electrically conductive trace (132) is configured to be altered in response to a second operation of the injection device (102) different than the first operation of the injection device (102).

8. The sensor assembly (104) of claim 7, wherein the first operation of the injection device (102) includes removal of a cap or a cover of the injection device (102), and wherein the second operation of the injection device (102) includes actuation of a button of the injection device (102) or a deployment of a needle shield of the injection device (102).

9. The sensor assembly (104) of claim 1, wherein the disposable label (130) includes at least one sensor (137), wherein the at least one sensor (137) includes at least one of the following: an optical sensor, a temperature sensor, a force sensor, or any combination thereof, and wherein the at least one sensor (137) is electrically coupled to the electrically conductive trace (132).

## Patentansprüche

1. Eine Sensoranordnung (104) für eine Injektionsvorrichtung (102), die Folgendes umfasst:
ein Einwegetikett (130), das so konfiguriert ist, dass es an der Injektionsvorrichtung (102) angebracht werden kann, wobei das Einwegetikett (130) Folgendes umfasst:
eine elektrisch leitende Spur (132), wobei ein erster Abschnitt der elektrisch leitenden Spur (132) so konfiguriert ist, dass er sich als Reaktion auf eine erste Betätigung der Injektionsvorrichtung (102) ändert;
ein wiederverwendbares Gehäuse (150), das abnehmbar mit dem Einwegetikett (130) verbunden ist, wobei das wiederverwendbare Gehäuse (150) Folgendes umfasst:
einen Prozessor (152), der elektrisch mit der elektrisch leitenden Spur (132) gekoppelt ist, wobei der Prozessor (152) konfiguriert ist, um als Reaktion auf eine Änderung des ersten Abschnitts der elektrisch leitenden Spur (132) aktiviert zu werden; und
eine drahtlose Kommunikationsvorrichtung (154), die so konfiguriert ist, dass sie drahtlos Informationen, die mit der Änderung des ersten Abschnitts der elektrisch leitenden Spur (132) verbunden sind, an eine Computervorrichtung übermittelt,
**dadurch gekennzeichnet, dass** der Prozessor (152) abnehmbar elektrisch mit der elektrisch leitenden Spur (132) gekoppelt ist und wobei das Einwegetikett (130) eine Vielzahl von ersten leitenden Anschlussflächen (702) enthält, die elektrisch mit der elektrisch leitenden Spur (132) gekoppelt sind, wobei das wiederverwendbare Gehäuse (150) eine Vielzahl von Federkontakten (704) aufweist, die elektrisch mit dem Prozessor (152) gekoppelt sind und abnehmbar elektrisch mit der Vielzahl von ersten leitenden Anschlussflächen (702) gekoppelt sind, und wobei das wiederverwendbare Gehäuse (150) eine starre, verformbare Klammer (706) aufweist, die so konfiguriert ist, dass sie an einer Außenfläche der Injektionsvorrichtung (102) befestigt werden kann.

2. Sensoranordnung (104) nach Anspruch 1, wobei das Einwegetikett (130) eine Klebeschicht (136) enthält.

3. Sensoranordnung (104) nach Anspruch 1, wobei das Einwegetikett (130) eine Batterie (138) enthält.

4. Die Sensoranordnung (104) nach Anspruch 1, wobei das wiederverwendbare Gehäuse (150) eine Batterie (138) enthält.

5. Sensoranordnung (104) nach Anspruch 1, wobei das Einwegetikett (130) eine weitere elektrisch leitende Spur (133) aufweist, wobei ein erster Abschnitt der weiteren elektrisch leitenden Spur (133) so konfiguriert ist, dass er als Reaktion auf eine zweite Betätigung der Injektionsvorrichtung (102), die sich von der ersten Betätigung der Injektionsvorrichtung (102) unterscheidet, geändert wird, und wobei der Prozessor (152) abnehmbar elektrisch mit der weiteren elektrisch leitenden Spur (133) verbunden ist.

6. Sensoranordnung (104) nach Anspruch 5, wobei die erste Betätigung der Injektionsvorrichtung (102) das Entfernen einer Kappe oder einer Abdeckung der Injektionsvorrichtung (102) umfasst, und wobei die zweite Betätigung der Injektionsvorrichtung (102) die Betätigung eines Knopfes der Injektionsvorrichtung (102) oder das Ausfahren eines Nadelschutzes der Injektionsvorrichtung (102) umfasst.

7. Sensoranordnung (104) nach Anspruch 1, wobei ein zweiter Abschnitt der elektrisch leitenden Spur (132) so konfiguriert ist, dass er sich als Reaktion auf eine zweite Betätigung der Injektionsvorrichtung (102), die sich von der ersten Betätigung der Injektionsvorrichtung (102) unterscheidet, ändert.

8. Sensoranordnung (104) nach Anspruch 7, wobei die erste Betätigung der Injektionsvorrichtung (102) das Entfernen einer Kappe oder einer Abdeckung der Injektionsvorrichtung (102) einschließt, und wobei die zweite Betätigung der Injektionsvorrichtung (102) die Betätigung eines Knopfes der Injektionsvorrichtung (102) oder das Ausfahren eines Nadelschutzes der Injektionsvorrichtung (102) einschließt.

9. Sensoranordnung (104) nach Anspruch 1, wobei das Einwegetikett (130) mindestens einen Sensor (137) aufweist, wobei der mindestens eine Sensor (137) mindestens einen der Folgenden aufweist: einen optischen Sensor, einen Temperatursensor, einen Kraftsensor oder eine beliebige Kombination davon, und wobei der mindestens eine Sensor (137) elektrisch mit der elektrisch leitenden Spur (132) gekoppelt ist.

## Revendications

1. Ensemble capteur (104) pour un dispositif d'injection (102) comprenant :
une étiquette jetable (130) configurée pour être fixée au dispositif d'injection (102), l'étiquette jetable (130) comprenant :
une piste électroconductrice (132), dans lequel une première partie de la piste électroconductrice (132) est configurée pour être altérée en réponse à une première opération du dispositif d'injection (102) ;
un boîtier réutilisable (150) relié de manière amovible à l'étiquette jetable (130), le boîtier réutilisable (150) comprenant :
un processeur (152) couplé électriquement à la piste électroconductrice (132), dans lequel le processeur (152) est configuré pour être activé en réponse à une altération de la première partie de la piste électroconductrice (132) ; et
un dispositif de communication sans fil (154) configuré pour transmettre sans fil, à un dispositif informatique, des informations associées à l'altération de la première partie de la piste électroconductrice (132),
**caractérisé en ce que**
le processeur (152) est couplé électriquement de manière amovible à la piste électroconductrice (132) et dans lequel l'étiquette jetable (130) comprend une pluralité de premiers plots conducteurs (702) couplés électriquement à la piste électroconductrice (132), dans lequel le boîtier réutilisable (150) comprend une pluralité de contacts à ressort (704) couplés électriquement au processeur (152) et couplés électriquement de manière amovible à la pluralité de premiers plots conducteurs (702), et dans lequel le boîtier réutilisable (150) comprend une pince déformable rigide (706) configurée pour se fixer sur une surface extérieure du dispositif d'injection (102).

2. Ensemble capteur (104) de la revendication 1, dans lequel l'étiquette jetable (130) comprend une couche adhésive (136).

3. Ensemble capteur (104) de la revendication 1, dans lequel l'étiquette jetable (130) comprend une batterie (138).

4. Ensemble capteur (104) de la revendication 1, dans lequel le boîtier réutilisable (150) comprend une batterie (138).

5. Ensemble capteur (104) de la revendication 1, dans lequel l'étiquette jetable (130) comprend une piste électroconductrice supplémentaire (133), dans lequel une première partie de la piste électroconductrice supplémentaire (133) est configurée pour être altérée en réponse à une seconde opération du dispositif d'injection (102), différente de la première opération du dispositif d'injection (102), et dans lequel le processeur (152) est couplé électriquement de manière amovible à la piste électroconductrice supplémentaire (133).

6. Ensemble capteur (104) de la revendication 5, dans lequel la première opération du dispositif d'injection (102) comprend le retrait d'un capuchon ou d'un couvercle du dispositif d'injection (102) et dans lequel la seconde opération du dispositif d'injection (102) comprend l'activation d'un bouton du dispositif d'injection (102) ou un déploiement d'une protection d'aiguille du dispositif d'injection (102).

7. Ensemble capteur (104) revendication 1, dans lequel une seconde partie de la piste électroconductrice (132) est configurée pour être altérée en réponse à une seconde opération du dispositif d'injection (102) différente de la première opération du dispositif d'injection (102).

8. Ensemble capteur (104) de la revendication 7, dans lequel la première opération du dispositif d'injection (102) comprend le retrait d'un capuchon ou d'un couvercle du dispositif d'injection (102) et dans lequel la seconde opération du dispositif d'injection (102) comprend l'activation d'un bouton du dispositif d'injection (102) ou un déploiement d'une protection d'aiguille du dispositif d'injection (102).

9. Ensemble capteur (104) de la revendication 1, dans lequel l'étiquette jetable (130) comprend au moins un capteur (137), dans lequel l'au moins un capteur (137) comprend au moins l'un de ce qui suit : un capteur optique, un capteur de température, un capteur de force ou une combinaison quelconque de ceux-ci, et dans lequel l'au moins un capteur (137) est couplé électriquement à la piste électroconductrice (132).
